# EUROPEAN PATENT SPECIFICATION

(11) **EP 0 446 605 B1**
(45) Date of publication and mention of the grant of the patent: **27.09.1995**
(21) Application number: 91101544.4
(22) Date of filing: 05.02.1991
(51) Int. Cl.: A61M 1/16, A61M 5/36

(54) **System for detecting the passage of air bubbles in the drip attachment of a dialysis unit**
Detektionssystem von Luftblasen in einer Tropfkammer einer Dialyse-Einheit
Système de détection de bulles d'air dans la chambre à goutte d'une unité de dialyse

(30) Priority: 08.02.1990 IT 333690
(43) Date of publication of application: 18.09.1991
(73) Proprietor: BELLCO S.p.A., I-41037 Mirandola (IT)
(72) Inventor: Cianciavicchia, Domenico, I-64040 Cavuccio (IT); Pradelli Alessandro, I-41034 Finale Emilia (IT)
(74) Representative: Boggio, Luigi

(56) References cited:
- EP-A- 0 181 272
- DE-A- 3 720 664
- US-A- 4 487 601

## Description

The present invention relates to a system for detecting the passage of air bubbles in the drip attachment of a dialysis unit.

On such units, and particularly along the line carrying the patient's blood or a physiological solution, the health of the patient depends on detecting the passage of air bubbles and activating alarm signals for cutting off the blood or solution supply when the air bubbles through the system exceed a predetermined threshold. The patent DE 3720664 relates a system which comprises an ultrasonic wave transmitter and receiver; a body with seats for a drip attachment, the transmitter and the receiver; and an electronic circuit for processing the signal from the receiver, which is proportional to the acoustic signal generated by the transmitter and to the attenuation produced by the passage of air bubbles. In the detection system employed on current units, the electronic circuit also comprises the signal from the receiver with a predetermined threshold value and, depending on the result, supplies an alarm signal and shuts down the pump supplying the fluid to the patient.

The above system presents several major drawbacks.

In particular, the threshold value activating the alarm signal is determined empirically, so that only crossover of the threshold, and not the actual size of the air bubbles, is detected. Also, the sensitivity of the system is affected by the manner in which the transmitter and receiver are fitted inside their respective seats, and by the difference in contact between the walls of the drip attachment and the transmitter and receiver when, as is the case for each treatment, the drip attachment is changed. A substantial difference may therefore exist between the alarm signals of different treatments, so that manufacturers tend to establish a high threshold value. In so doing, however, the system fails to detect any air bubbles below the set threshold, but which may be equally harmful to the patient.

The aim of the present invention is to provide a system for detecting the passage of air bubbles in the drip attachment of a dialysis unit, designed to overcome the aforementioned drawbacks.

With this aim in view, according to the present invention, there is provided a system for detecting the passage or air bubbles in the drip attachment of a dialysis unit, comprising:
a generator for generating a first alternating voltage signal;
a fist member installed next to said drip attachment, for converting said first signal into an acoustic signal through said drip attachment;
a second member installed next to said drip attachment and in line with said first member, for converting said acoustic signal into a second alternating voltage signal;
a first electronic circuit for amplifying said second signal by a gain which varies according to said second signal so as to produce a third alternating voltage signal of constant amplitude, and for converting said third signal into a fourth electric signal which, in the absence of air bubbles in said drip attachment, presents only a continuous component, and, in the presence of air bubbles, also presents a transient component produced by the air bubbles attenuating said signal in proportion to the volume of the air bubbles;
characterised by the fact it comprises:
an electric network for eliminating said continuous component in said fourth signal and letting through said transient component;
a second electronic circuit for converting said transient component into a fifth electric signal corresponding to a bubble alarm signal;
an electronic unit for receiving said fifth signal and shutting down all the devices on said unit for arresting the dialysis treatment; and
a body having a first seat housing said drip attachment,
and in which are formed diametrically-opposed second seats for said members.

A preferred, non-limiting embodiment of the present invention will be described by way of example with reference to the accompanying drawings, in which:
Fig.1 shows a view in perspective of a dialysis unit;
Fig.2 shows a larger-scale plan view of part of the Fig.1 unit;
Fig.3 shows an electronic circuit diagram of a system for detecting the passage of air bubbles and applied to the Fig.1 unit.

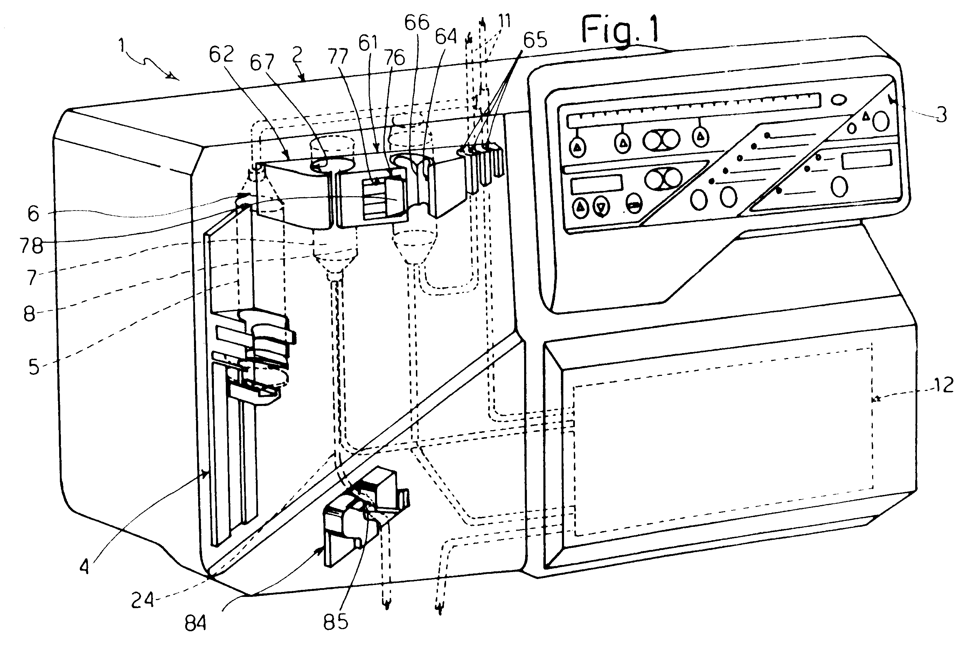

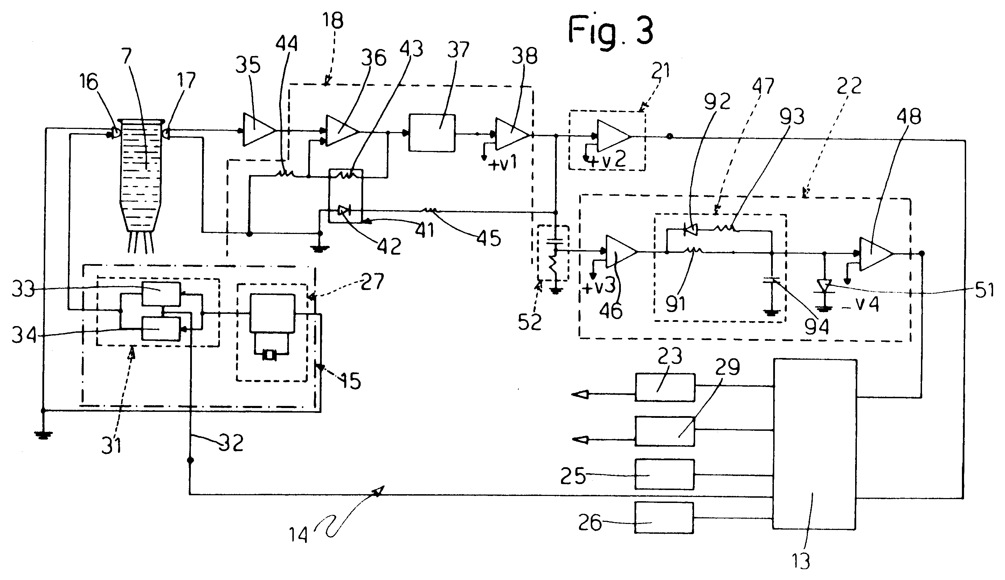

Number 1 in Fig.1 indicates a dialysis unit comprising a frame 2 housing known dialysis treatment devices. The front face of frame 2 supports a control panel 3; a body 4 supporting a heparin pump 5; and a body 6 supporting a drip attachment 7, a collecting tank 8, and conduits 11 of a known hydraulic system employed for said treatment. Below panel 3, frame 2 presents a seat for the pump unit 12 of said hydraulic system. Heparin pump 5, drip attachment 7, tank 8, conduits 11 and pump unit 12 are all of known type and therefore shown schematically by dotted lines. Unit 1 also comprises an electronic unit 13 (Fig.3) for controlling all the devices (electric motors, pump unit 12, sensors, safety devices, etc.) housed inside frame 2, and to which are connected all the controls, displays, alarm indicators, etc. on control panel 3. Number 14 in Fig.3 indicates an electronic system for detecting the passage of air bubbles through drip attachment 7. System 14 is housed inside frame 2 and comprises:
a transmitting block 15;
a transmitting sensor 16;
a receiving sensor 17;
an automatic voltage signal gain control block 18;
a first comparing block 21 for a drip attachment fluid level alarm;
a second comparing block 22 for an alarm indicating the passage of air bubbles through drip attachment 7;
said unit 13;
an actuating member 23 for arresting pump unit 12;
an actuating member 29 for closing conduit 24 feeding said fluid back into the patient's vein; and
an alarm light 25 and acoustic alarm 26, preferably installed on control panel 3.

Block 15 comprises a block 27 for generating square-wave voltage signals and preferably consisting of a 2 MHz quartz oscillator; and a switch block 31 connected or disconnected by a logic (test) signal transmitted over line 32 by unit 13. When disconnected, block 31 allows the signal generated by block 27 to be applied fully to transmitting sensor 16. When connected, on the other hand, block 31 provides for partial (preferably two-decibel) attenuation of said signal from block 27. For a clearer understanding of the operation of block 31, this is shown schematically in Fig.3 in two blocks 33 and 34.

Depending on said test signal from unit 13, the signal from block 27 is supplied either to block 33, which provides for no attenuation, or to block 34, which provides for preferably two-decibel attenuation. The advantages afforded by block 31 and said logic test signal will become apparent later on.

Transmitting sensor 16 is an ordinary piezoelectric transducer for converting an alternating electric signal into an ultrasonic acoustic signal. Similarly, receiving sensor 17 is a piezoelectric transducer for converting an acoustic signal into an alternating electric signal. Series connected to receiving sensor 17, system 14 comprises a two-stage signal amplifying circuit, the first stage consisting of a constant-gain amplifying block 35 for amplifying, e.g. three times, the signal transmitted by receiving sensor 17, and the second stage consisting of block 18. Block 18 comprises, in series, an amplifying block 36; a rectifying block 37 for converting the incoming alternating signal into a continuous positive signal; and an error amplifying block 38 for comparing the output signal from block 37 with a continuous positive reference voltage +V1 and amplifying the resulting signal. For amplifying block 36, block 18 also comprises a feedback loop for varying the gain effected by amplifying block 18, and which presents an optocoupler 41 consisting of a photodiode 42 and photoresistor 43.

In particular, block 18 comprises:
amplifying block 36 having a first input connected to the output of amplifying block 35; its output connected to the input of rectifying block 37; and a second input grounded via resistor 44 and connected to said output via photoresistor 43;
rectifying block 37 having its output connected to a first input of amplifying block 38;
amplifying block 38 having a second input connected to said voltage +V1, and its output connected to said ground via resistor 45 and photodiode 42.

Comparing block 21 presents a first input connected to the output of amplifying block 38; a second input connected to a continuous reference voltage +V2; and its output connected to one input of unit 13.

Comparing block 22 comprises, in series, a comparing block 46; an integrating block 47; a comparing block 48; and a diode 51 on a grounded line branching off that connecting integrating block 47 and comparing block 48. Comparing block 46 presents a first input connected via a standard C/R network 52 to the output of amplifying block 38; a second input connected to a continuous reference voltage +V3; and its output connected to the input of integrating block 47. Comparing block 48 presents a first input connected to the output of integrating block 47; a second input connected to a continuous reference voltage -V4; and its output connected to one input of unit 13.

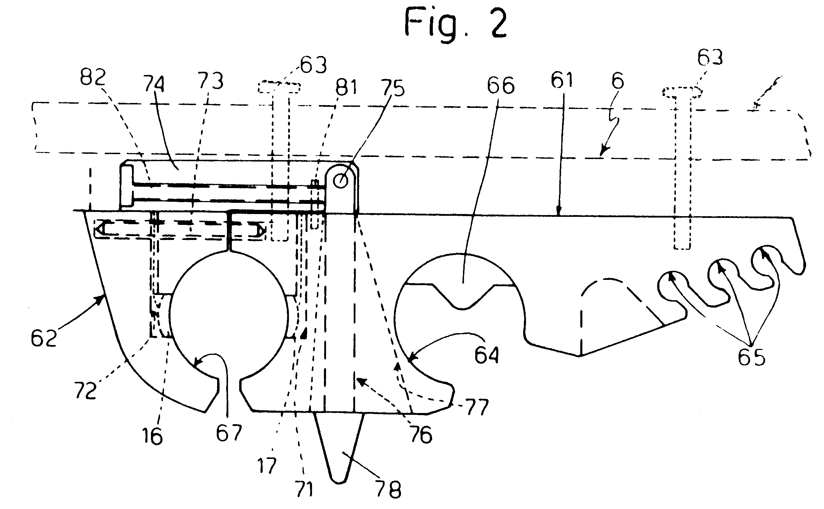

As shown in Fig.2, body 6 comprises a fixed portion 61 made of plastic material, and a smaller portion 62 also made of plastic and designed to move between two positions wherein drip attachment 7 is locked and released respectively. Portion 61 presents a flat rear face secured by screws 63 (shown by the dotted line) to the front face of frame 2; a lateral face in which is formed a semicylindrical recess having a vertical axis; and a front face in which are formed, in series, a vertical-axis seat 64 for collecting tank (, and vertical-axis seats 65 for conduits 11. The thickness of portion 61 between the front and rear faces decreases between the portions in which seat 64 and seats 65 are formed. Seat 64 presents a projection 66 designed to mate with a corresponding recess on tank 8 and so prevent the wrong type of tank from being inserted inside seat 64.

Portion 62 presents a rear face coplanar with the rear face of portion 61; and a lateral face facing that of portion 61. The lateral face of portion 62 also presents a semicylindrical recess with a vertical axis, which, together with the recess in the lateral face of portion 61, defines a cylindrical seat 67 for drip attachment 7. Seats 64, 65 and 67 are all open at the front. The recesses in the lateral faces of portions 61 and 62 present respective recesses 71 and 72 formed on a level with each other and in which are housed receiving sensor 17 and transmitting sensor 16 respectively. In use, sensors 16 and 17 are embedded in a layer of epoxy resin having a semicylindrical surface, so as to adhere to the cylindrical surface of drip attachment 7. Portion 62 is designed to travel in relation to portion 61 between two positions: a first close contact position, wherein it defines a grip for drip attachment 7 and presses sensors 16 and 17 against the lateral surface of the same; and a second position wherein it is located a given distance from portion 61, thus enabling removal of drip attachment 7 from seat 67. Portion 62 slides on two fixed pins 73 fitted inside respective seats formed to the side of said recess in said lateral face of portion 61, and sliding in respective dead holes formed to the side of said recess in said lateral face of portion 62. The rear face of portion 62 supports an integral plastic bar 74 extending parallel to the rear face of portion 62 and terminating at a given portion of the rear face of portion 61. The end of bar 74 at the rear face of portion 61 presents a vertical pin 75 on which pivots a first end of a lever 76 inside a through opening 77 between the front and rear faces of portion 61 and formed between the lateral face of portion 61 and the portion in which seat 64 is formed. The second end 78 of lever 76 extends beyond the front face of portion 61. A fixed pin 81 is fitted inside the rear face of portion 61, and extends inside bar 74 where it is connected integral with a first end of a preloaded spring 82, the second end of which is secured to the opposite end of bar 74 to that fitted with pin 75. Spring 82 counteracts displacement of portion 62 between said first and second positions.

When energized by an alternating voltage signal with, for example, a square wave of 2 MHz frequency, transmitting sensor 16 generates acoustic waves which travel through the soft wall of drip attachment 7 to the fluid inside. Through said fluid, the acoustic wave is transmitted to sensor 17 at the terminals of which a voltage signal of a given value is produced. When the fluid level in drip attachment 7 is below the axis of sensors 16 and 17, no acoustic wave is transmitted to sensor 17, with the result that no signal is produced at the terminals of the same. The absence of said signal is detected by the circuit, which accordingly supplies a level alarm signal. In the presence of fluid inside drip attachment 7, an air bubble across the axis of sensors 16 and 17 attenuates the signal received by sensor 17, which attenuation is proportional to the volume of the air bubble and provides for producing a proportional bubble alarm signal. As already stated, oscillating block 27 generates a square wave signal of, for example, 5 V amplitude, which is sent to attenuating block 31 enabled by a logic signal. In the event of attenuation of the electric signal corresponding to approximately two-decibel attenuation of the acoustic signal produced by sensor 16, on the basis of the amplitude of the signal produced by block 27 in sensor 16, an acoustic level equal to a fraction of the nominal level is produced. Attenuation of the acoustic level through the fluid, as would be produced in the presence of a given quantity of air, may thus be simulated electronically, for checking operation of the alarms at regular intervals during the dialysis treatment. Said artificial attenuation, in fact, simulates the presence of a given quantity of air and, therefore, a given bubble alarm signal. Should all or even part of said alarm signal fail to be received by unit 13, this indicates a fault on the circuit, with the result that unit 13 arrests operation of unit 1, particularly supply of the fluid along conduit 24, by activating a grip device 84 as shown in Fig.1. Conduit 24 runs through said grip device 84, which presents a tooth 85 controlled by actuating member 29, for enabling or cutting off fluid supply along conduit 24.

As already stated, block 18 constitutes one amplifying stage of the output signal from amplifying block 35, the gain of which varies according to the incoming signal. Consequently, the output signal from amplifying block 36 presents a substantially constant amplitude, even in the event of inconsistent contact between sensors 16 and 17 and drip attachment 7, which inconsistency is inevitable in a component, such as drip attachment 7, that is changed between treatments and presents different design features depending on the make. The system according to the present invention may therefore be said to adapt automatically, between treatments, to any difference in mechanical contact between sensors 16 and 17 and drip attachment 7, while at the same time maintaining a high degree of sensitivity in detecting the presence of air bubbles through attachment 7.

Block 18 is designed to operate between two threshold values corresponding to two-decibel attenuation of the acoustic level of sensor 16. This is based on practical experience, which has shown the signal at the terminals of sensor 17 to be a sinusoidal signal with a peak-peak amplitude ranging from 10 to 100 millivolts, allowing for both filtration by the fluid and frequent variations in contact between sensors 16 and 17 and drip attachment 7. In actual use, maximum gain by both amplifying stages is approximately 300 times, which would be excessive for block 36 alone. Block 35 therefore provides for increasing the signal 3 times, and block 36 for a maximum gain of 100 times. Optocoupler 41 provides for automatically varying the gain of amplifying block 36. In fact, an increase in the excitation current of photodiode 42 produces a proportional reduction in the resistivity of photoresistor 43 and, consequently, the gain of block 36. Photodiode 42 is controlled by block 38, which is designed (assuming the design features described above by way of example) to increase the continuous voltage signal 100 times. Instant by instant, block 38 compares the voltage output signal from block 37 with voltage +V1, the value of which is so selected as to obtain a peak-peak voltage of 3 V at the output of block 36 when this, of course, operates between said threshold values. The higher the output signal from block 37 is in relation to +V1, the higher the excitation current of photodiode 42 will be, thus reducing the gain of block 36 and vice versa. It should be pointed out that photodiode 42 is controlled by electrical currents between the ground connection and the output of block 38, which normally presents (assuming the design features described above) a negative voltage ranging from 0 to -10 V. When the amplitude of the output signal from block 37 is below +V1, block 38 operates in the voltage range close to zero, so as to fully disable photodiode 42. If the resulting increase in the resistivity of photoresistor 43 fails to produce the gain required for "locking in" block 36 (i.e. operating it within its specified threshold values), the feedback loop is open, and the output of block 38 presents a voltage tending towards +15 V. The positive voltage at the output of block 38 is compared with +V2, the result of which comparison produces said level alarm signal indicating that the fluid level in drip attachment 7 is below the axis of sensors 16 and 17. When this occurs, the acoustic signal is attenuated in excess of the design value, thus causing a drastic reduction in the amplitude of the output signal from amplifying block 36. This also results in a reduction of the continuous component of the output signal from rectifying block 37, which in turn produces a high error voltage at the input of amplifying block 38. In view of the high increase produced by amplifying block 38, the output voltage from the same will tend to rise to compensate for the variation in the gain of block 36. In actual fact, however, the output of block 38 becomes saturated, thus departing from the linearity presented under normal operating conditions, and the feedback loop of block 36 is opened. As already stated, saturation of block 38 produces a signal which, appropriately clipped by block 21, is sent to unit 13 for arresting pump unit 12, shutting off conduit 24, and activating the level alarm and indicator light.

In the event of air bubbles crossing the axis of sensors 16 and 17, the acoustic signal is attenuated only temporarily, by a smaller amount as compared with the level alarm, and in proportion to the volume of the bubble. As the phenomenon (air bubble passage) is too brief for the output of block 38 to become saturated, the continuous voltage at the output of block 38 will present a transient component constituting said signal proportional to the size of the bubble. Network 52 eliminates the continuous component (which serves solely for locking in the feedback loop) and only lets through the transient component, which is sent to block 46 for generating a clipped pulse proportional to the size of the bubble. Passage of a bubble across the axis of sensors 16 and 17 therefore only produces a positive voltage peak at the output of block 38, with no noticeable change in the continuous component of the voltage at said output, and the feedback loop remains locked in. The time constant of network 52 is so designed as to let through considerably long signals, usually of over 10 milliseconds, with no noticeable attenuation. Block 46 compares the output signal from network 52 with +V3 to give a clipped signal of the same length as the incoming signal and with an amplitude usually ranging from +15 to -15 V. Block 46 is so selected as to enable pulses to be generated even in the presence of short incoming signals. Generally speaking, the block 46 employed provides for generating pulses of over 100 microseconds, and presents an output of +15 V under normal operating conditions.

Block 47 comprises two parallel branches, the first of which presents a resistor 91, and the second of which presents a diode 92 and a resistor 93 of a lower value than resistor 91. Both branches are grounded via condenser 94. Block 47 provides for asymmetrically integrating the signal from block 46, to produce, at the terminals of condenser 94, a voltage proportional to the length of the signal from block 46, when a predetermined time period (greater than the discharge time of condenser 94) elapses between one signal and the next, or proportional to the sum of pulses shorter than said time period, providing said pulses are very close together. In the latter case, block 47 provides for counting the bubbles through drip attachment 7, the passage of a number of successive small-volume bubbles being equated to that of a single larger-volume bubble. In the presence of air bubbles, condenser 94 is discharged via resistors 91 and 93 for the duration of the pulse generated by block 46, and is discharged via resistor 93 when the pulse ceases. By virtue of the differing values of resistors 91 and 93, the charging time of condenser 94 is greater than the discharging time. Consequently, in the presence of a series of air bubbles, condenser 94 is discharged increasingly until the voltage at its terminals equals -V4, thus resulting, in block 48, in the generation of a pulse constituting said bubble alarm signal. The value of -V4 therefore determines the operating threshold of block 48, i.e. generation of a pulse which, via unit 13, arrests unit 1 and activates the bubble alarm and indicator light. By varying the value of -V4, e.g. by means of a potentiometer, the operating threshold of block 48 is varied in relation to the integrating constant of block 47. That is, an increase in the absolute value of -V4 increases the delay produced by block 47, so that a greater number of air bubbles is required for generating the bubble alarm signal. If -V4 is set close to 0 volts, block 48 generates an alarm signal even in the presence of minute air bubbles.

The advantages of the present invention will be clear from the foregoing description.

In particular, the system according to the present invention provides for generating alarm signals in the presence of both a low fluid level and air bubbles in drip attachment 7. The system adapts automatically, while at the same time maintaining a high degree of sensitivity, to drip attachments 7 of different design and, therefore, to differing contact between attachment 7 and sensors 16 and 17, thus enabling the alarm signal threshold to be predetermined, by adjusting the value of -V4, regardless of the type of drip attachment employed. Moreover, the system provides, during treatment, for periodically testing the alarm signals by simulating a predetermined attenuation of the acoustic signal transmitted through drip attachment 7. In other words, the system provides for safety conditions defined, not empirically by the operator, but automatically, thus ensuring maximum safety of the patient. Finally, body 6 is so designed as to enable troublefree insertion and removal of drip attachment 7, by operating end 78 of lever 76 against the action of spring 82, and troublefree assembly of most of the components changed between treatments.

To those skilled in the art it will be clear that changes may be made to the system as described and illustrated herein without, however, departing from the scope of the present invention.

## Claims

1. A system for detecting the passage or air bubbles in the drip attachment (7) of a dialysis unit (1), comprising:
a generator (27) for generating a first alternating voltage signal;
a first member (16) installed next to said drip attachment (7), for converting said first signal into an acoustic signal through said drip attachment (7);
a second member (17) installed next to said drip attachment (7) and in line with said first member (16), for converting said acoustic signal into a second alternating voltage signal;
a first electronic circuit (18) for amplifying said second signal by a gain which varies according to said second signal so as to produce a third alternating voltage signal of constant amplitude, and for converting said third signal into a fourth electric signal which, in the absence of air bubbles in said drip attachment (7), presents only a continuous component, and, in the presence of air bubbles, also presents a transient component produced by the air bubbles attenuating said signal in proportion to the volume of the air bubbles;
characterised by the fact it comprises:
an electric network (52) for eliminating said continuous component in said fourth signal and letting through said transient component;
a second electronic circuit (22) for converting said transient component into a fifth electric signal corresponding to a bubble alarm signal;
an electronic unit (13) for receiving said fifth signal and shutting down all the devices on said unit (1) for arresting the dialysis treatment; and
a body (6) having a first seat (67) housing said drip attachment (7), and in which are formed diametrically-opposed second seats (72 and 71) for said members (16 and 17).

2. A system as claimed in Claim 1, characterised by the fact that said first electronic circuit (18) comprises:
a first amplifying block (36) for amplifying said second signal;
a feedback loop for said first amplifying block (36), having an electronic device (41) for automatically controlling the gain of said first amplifying block (36) and producing, at the output of the same, said third alternating voltage signal of constant amplitude;
a rectifying block (37) for converting said third signal into a fourth continuous voltage signal; and
a second amplifying block (38) for comparing said fourth signal with a first reference voltage (+V1), generating said fourth continuous voltage signal proportional to the result of said comparison, and controlling said device (41) via said fourth signal.

3. A system as claimed in Claim 2, characterised by the fact that said electronic device (41) comprises an optocoupler having a photoresistor (43) and a photodiode (42).

4. A system as claimed in Claim 3, characterised by the fact that:
said first amplifying block (36) presents a first input for receiving said second signal; an output connected to the input of said rectifying block (37); and a second input grounded via a first resistor (44) and connected to its output via said photoresistor (43);
said rectifying block (37) presents its output connected to a first input of said second amplifying block (38); and
said second amplifying block (38) presents a second input connected to said first reference voltage (+V1); and its output connected to said ground via a second resistor (45) and said photodiode (42).

5. A system as claimed in at least one of the foregoing Claims, characterised by the fact that said second circuit (22) comprises, in series, a first comparing block (46), an integrating block (47) and a second comparing block (48).

6. A system as claimed in Claim 5, characterised by the fact that:
said first comparing block (46) presents a first input connected via said network (52) to the output of said second amplifying block (38); a second input connected to a second continuous reference voltage (+V3); and its output connected to the input of said integrating block (47);
said second comparing block (48) presents a first input connected to the output of said integrating block (47); a second input connected to a third continuous reference voltage (-V4); and its output connected to an input of said unit (13).

7. A system as claimed in Claim 6, characterised by the fact that said integrating block (47) comprises two parallel branches, the first of which presents a third resistor (91), and the second of which presents a diode (92) and a fourth resistor (93) having a lower value than said third resistor (91); both said branches being grounded via a condenser (94).

8. A system as claimed in Claim 7, characterised by the fact that said integrating block (47) provides for asymmetrically integrating the signal generated by said first comparing block (46), and for producing, at the terminals of said condenser (94), a voltage proportional to the length of the signal generated by said first comparing block (46) when a predetermined time period, greater than the discharge time of said condenser (94), elapses between one signal and the next, or proportional to the sum of pulses shorter than said time period, providing said pulses are very close together; said integrating block (47) providing, in the latter case, for counting the bubbles through said drip attachment (7), the passage of a number of successive small-volume bubbles being equated to that of a single larger-volume bubble.

9. A system as claimed in at least one of the foregoing Claims from 2 to 8, characterised by the fact that it comprises a first comparing block (21) for comparing said fourth signal with a fourth reference voltage (+V2), so that, in the event of the fluid level in said drip attachment (7) being below the axis of said members (16 and 17) thus determining a value close to zero of said fourth signal, said fourth signal, for controlling said device (41), tends towards a high value which, when compared, defines at the output of said third comparing block (21) a sixth signal which is sent to said unit (13) for arresting the dialysis treatment.

10. A system as claimed in any one of the foregoing Claims, characterised by the fact that it comprises, upstream from said first member (16), a switch block (31) connected or disconnected by a logic (test) signal transmitted over a line (32) from said unit (13); said switch block (31), when disconnected, enabling said first signal to be supplied fully to said first member (16), and, when connected, attenuating a predetermined portion of said first signal for simulating the presence of a predetermined quantity of air and, therefore, a predetermined bubble alarm signal, for detecting any faulty system components and enabling said unit (13) to arrest operation of said dialysis unit (1).

11. A system as claimed in any one of the foregoing Claims, characterised by the fact that it comprises a first actuator (23) for enabling a pump unit (12) forming part of the hydraulic circuit of said unit (1); a second actuator (29) for enabling fluid supply from said drip attachment (7) via a grip device (84); and luminous and/or acoustic alarm indicators (25 and 26); said actuators (23 and 29) and said indicators (25 and 26) being controlled by said unit (13).

12. A system as claimed in any one of the foregoing Claims, characterised by the fact that said body (6) is formed in two parts, a first (61) of which is fixed, and a second (62) of which is designed to move, by virtue of a lever operated manually against the action of a spring (82), between a first position wherein, by virtue of said spring (82), said drip attachment (7) is gripped between said parts (61 and 62), and a second position wherein said second part (62) is located a predetermined distance from the other, thus enabling removal of said drip attachment (7) from said first seat (67).

13. A system as claimed in Claim 12, characterised by the fact that said parts (61 and 62) present a respective lateral face in which is formed a respective semicylindrical recess; said recesses defining said first seat (67).

14. A system as claimed in Claim 13, characterised by the fact that said first part (61) presents a third seat (64) for housing a tank (8); and recesses (65) for supporting conduits (11); said third seat (64) presenting an inner projection (66) only enabling insertion of tanks (8) having a recess compatible with said projection (66).

## Patentansprüche

1. System zum Erfassen des Durchgangs von Luftblasen in der Tropfeinrichtung (7) einer Dialyseeinheit (1) mit:
einem Generator (27), der ein erstes Wechselspannungssignal erzeugt;
einem ersten Teil (16), das in der Nähe der Tropfeinrichtung (7) befestigt ist und das erste Signal in ein akustisches Signal durch die Tropfeinrichtung (7) umwandelt;
einem zweiten Teil (17), das in der Nähe der Tropfeinrichtung (7) und auf einer Linie mit dem ersten Teil (16) befestigt ist und das akustische Signal in ein zweites Wechselspannungssignal umwandelt;
einer ersten elektronischen Schaltung (18), die das zweite Signal um einen Verstärkungsfaktor verstärkt, welcher sich in Übereinstimmung mit dem zweiten Signal ändert, um ein drittes Wechselspannungssignal einer konstanten Amplitude zu erzeugen und um das dritte Signal in ein viertes elektrisches Signal umzuwandeln, welches beim Nichtvorhandensein von Luftblasen in der Tropfeinrichtung (7) lediglich eine Gleichkomponente aufweist und beim Vorhandensein von Luftblasen ebenso eine Momentankomponente aufweist, die von den Luftblasen, die das Signal proportional zu dem Volumen der Luftblasen dämpfen, erzeugt wird;
dadurch gekennzeichnet, daß es aufweist:
ein elektrisches Netzwerk (52), das die Gleichkomponente in dem vierten Signal auslöscht und die Momentankomponente durchläßt;
eine zweite elektronische Schaltung (22), die die Momentankomponente in ein fünftes elektrisches Signal, das einem Blasen-Alarmsignal entspricht, umwandelt;
eine elektronische Einheit (13), die das fünfte Signal empfängt und zum Anhalten der Dialysebehandlung alle Vorrichtungen auf der Einheit (1) abschaltet; und
einen Körper (6), der eine erste Befestigung (67) aufweist, die die Tropfeinrichtung (7) unterbringt, und in welcher diametral gegenüberliegend zweite Befestigungen (72 und 71) für die Teile (16 und 17) ausgebildet sind.

2. System nach Anspruch 1, dadurch gekennzeichnet, daß die erste elektronische Schaltung (18) aufweist:
einen ersten Verstärkerblock (36), der das zweite Signal verstärkt;
eine Rückkopplungsschleife für den ersten Verstärkerblock (36), die eine elektronische Vorrichtung (41) aufweist, die den Verstärkungsfaktor des ersten Verstärkerblocks (36) automatisch steuert und an dem Ausgang des gleichen das dritte Wechselspannungssignal einer konstanten Amplitude erzeugt;
einen Gleichrichterblock (37), der das dritte Signal in ein viertes Gleichspannungssignal umwandelt; und
einen zweiten Verstärkerblock (38), der das vierte Signal mit einer ersten Referenzspannung (+V1) vergleicht, das vierte Gleichspannungssignal proportional zu dem Ergebnis des Vergleichs erzeugt und die Vorrichtung (41) über das vierte Signal steuert.

3. System nach Anspruch 2, dadurch gekennzeichnet, daß die elektronische Vorrichtung (41) einen Optokoppler aufweist, der einen Fotowiderstand (43) und eine Fotodiode (42) aufweist.

4. System nach Anspruch 3, dadurch gekennzeichnet, daß:
der erste Verstärkerblock (36) einen ersten Eingang, der das zweite Signal empfängt; einen Ausgang, der an den Eingang des Gleichrichterblocks (37) angeschlossen ist; und einen zweiten Eingang aufweist, der über einen ersten Widerstand (44) an Masse gelegt ist und über den Fotowiderstand (43) an seinen Ausgang angeschlossen ist;
der Ausgang des Gleichrichterblocks (37) an einen ersten Eingang des zweiten Verstärkerblocks (38) angeschlossen ist; und
ein zweiter Eingang des zweiten Verstärkerblocks (38) an die erste Referenzspannung (+V1) angeschlossen ist; und sein Ausgang über einen zweiten Widerstand (45) und die Fotodiode (42) an Masse gelegt ist.

5. System nach einem der vorhergehenden Ansprüche, dadurch gekennzeichnet, daß die zweite Schaltung (22) einen ersten Vergleichsblock (46), einen Integrierblock (47) und einen zweiten Vergleichsblock (48) in Serie aufweist.

6. System nach Anspruch 5, dadurch gekennzeichnet, daß:
der erste Vergleichsblock (46) einen ersten Eingang, der über das Netzwerk (52) an den Ausgang des zweiten Verstärkerblocks (38) angeschlossen ist; einen zweiten Eingang, der an eine zweite Referenzgleichspannung (+V3) angeschlossen ist; und seinen Ausgang aufweist, der an den Eingang des Integrierblocks (47) angeschlossen ist;
der zweite Vergleichsblock (48) einen ersten Eingang, der an den Ausgang des Integrierblocks (47) angeschlossen ist; einen zweiten Eingang, der an eine dritte Referenzgleichspannung (-V4) angeschlossen ist; und seinen Ausgang aufweist, der an einen Eingang der Einheit (13) angeschlossen ist.

7. System nach Anspruch 6, dadurch gekennzeichnet, daß der Integrierblock (47) zwei Parallelzweige aufweist, von denen der erste einen dritten Widerstand (91) aufweist und von denen der zweite eine Diode (92) und einen vierten Widerstand (93), der einen niedrigeren Wert als der dritte Widerstand (91) aufweist, aufweist; wobei beide Zweige über einen Kondensator (94) an Masse gelegt sind.

8. System nach Anspruch 7, dadurch gekennzeichnet, daß der Integrierblock (47) ein asymmetrisches Integrieren des Signals vorsieht, das von dem ersten Vergleichsblock (46) erzeugt wird, und an den Anschlüssen des Kondensators (94) eine Spannung erzeugt, die proportional zu der Länge des Signals ist, das von dem ersten Vergleichsblock (46) erzeugt wird, wenn eine vorbestimmte Zeitdauer, die größer als die Entladezeit des Kondensators (94) ist, zwischen einem Signal und dem nächsten verstreicht, oder die proportional zu der Summe von Pulsen ist, die kürzer als die Zeitdauer sind, wobei die Pulse sehr nahe zusammen geliefert werden; wobei der Integrierblock (47) im letzteren Fall zum Zählen der Blasen durch die Tropfeinrichtung (7) vorgesehen ist, wobei der Durchgang einer Anzahl von aufeinanderfolgenden Blasen eines kleinen Volumens dem einer einzigen Blase eines größeren Volumens gleichgesetzt ist.

9. System nach einem der vorhergehenden Ansprüche 2 bis 8, dadurch gekennzeichnet, daß sie einen ersten Vergleichsblock (21) aufweist, der das vierte Signal mit einer vierten Referenzspannung (+V2) vergleicht, so daß beim Auftreten des Flüssigkeitspegels in der Tropfeinrichtung (7), der sich unterhalb der Achse der Teile (16 und 17) befindet, somit ein Wert des vierten Signals nahe an Null bestimmt wird, wobei sich das vierte Signal zum Steuern der Vorrichtung (41) zu einem hohen Wert bewegt, welcher, wenn er verglichen wird, an dem Ausgang des dritten Vergleichsblocks (21) ein sechstes Signal definiert, welches zum Anhalten der Dialysebehandlung zu der Einheit (13) gesendet wird.

10. System nach einem der vorhergehenden Ansprüche dadurch gekennzeichnet, daß es stromaufwärts von dem ersten Teil (16) einen Schaltblock (31) aufweist, der von einem logischen (Test-) Signal, das über eine Leitung (32) aus der Einheit (13) übertragen wird, angeschlossen oder abgetrennt wird; wobei der Schaltblock (31), wenn er abgetrennt ist, ermöglicht, daß das erste Signal vollständig zu dem ersten Teil (16) geliefert wird, und wenn er angeschlossen ist, einen vorbestimmten Teil des ersten Signals zum Simulieren des Vorhandenseins einer vorbestimmten Luftmenge und deshalb eines vorbestimmten Blasen-Alarmsignals zum Erfassen aller fehlerhaften Systemkomponenten und zum Ermöglichen, daß die Einheit (13) einen Betrieb der Dialyseeinheit (1) anhält, dämpft.

11. System nach einem der vorhergehenden Ansprüche, dadurch gekennzeichnet, daß es eine erste Betätigungseinrichtung (23), die es einer Pumpeinheit (12) ermöglicht, einen Teil der hydraulischen Schaltung der Einheit (1) auszubilden; eine zweite Betätigungseinrichtung (29), die eine Flüssigkeitszufuhr aus der Tropfeinrichtung (7) über eine Klemmvorrichtung (84) ermöglicht; und leuchtende und/oder akustische Alarmanzeigeeinrichtungen (25 und 26) aufweist; wobei die Betätigungseinrichtungen (23 und 29) und die Anzeigeeinrichtungen (25 und 26) von der Einheit (13) gesteuert werden.

12. System nach einem der vorhergehenden Ansprüche dadurch gekennzeichnet, daß der Körper (6) in zwei Teilen ausgebildet ist, von denen ein erstes (61) fest ist und von denen ein zweites (62) so aufgebaut ist, daß es sich kraft eines Hebels, der manuell betätigt wird, gegen die Wirkung einer Feder (82) zwischen einer ersten Position, bei der kraft der Feder (82) die Tropfeinrichtung (7) zwischen die Teile (61) geklemmt ist und einer zweiten Position, in der sich das zweite Teil (62) in einem vorbestimmten Abstand von dem anderen befindet, bewegt, wodurch ein Entfernen der Tropfeinrichtung (7) aus der ersten Befestigung (67) ermöglicht wird.

13. System nach Anspruch 12, dadurch gekennzeichnet, daß die Teile (61 und 62) eine jeweilige Seitenfläche aufweisen, in welcher eine jeweilige halbzylindrische Aussparung ausgebildet ist; wobei die Aussparungen die erste Befestigung (67) definieren.

14. System nach Anspruch 13, dadurch gekennzeichnet, daß das erste Teil (61) eine dritte Befestigung (64), die einen Behälter (8) unterbringt; und Aussparungen (65) aufweist, die Rohrleitungen (11) tragen; wobei die dritte Befestigung (64) einen inneren Ansatz (66) aufweist, der lediglich ein Einführen eines Behälters (8) ermöglicht, der mit dem Ansatz (66) verträglich ist.

## Revendications

1. Système pour détecter le passage de bulles d'air dans la chambre à gouttes (7) d'une unité de dialyse (1) comprenant :
- un générateur (27) pour générer un premier signal de tension alternatif,
- un premier organe (16) installé près de la chambre à gouttes (7) pour convertir le premier signal en un signal acoustique à travers la chambre à gouttes (7),
- un deuxième organe (17) installé près de la chambre à gouttes (7) en ligne avec le premier organe (16), pour convertir le signal acoustique en un deuxième signal de tension alternatif,
- un premier circuit électronique (18) pour amplifier le deuxième signal d'un gain qui varie selon le deuxième signal afin de produire un troisième signal de tension alternatif d'amplitude constante, et pour convertir le troisième signal en un quatrième signal électrique qui, en l'absence de bulles d'air dans la chambre à gouttes (7), ne présente qu'une composante continue et en présence de bulles d'air, présente également une composante transitoire produite par les bulles d'air atténuant le signal en proportion au volume de ces bulles d'air,
caractérisé en ce qu'il comprend :
- un réseau électronique (52) pour éliminer la composante continue dans le quatrième signal et laisser passer la composante transitoire,
- un deuxième circuit électronique (22) pour convertir la composante transitoire en un cinquième signal électrique correspondant au signal d'alarme de bulles,
- une unité électronique (13) pour recevoir le cinquième signal et couper tous les dispositifs sur l'unité (1) afin d'arrêter le traitement de dialyse, et
- un corps (6) présentant un premier logement en forme de support (67) pour la chambre à gouttes (7), et dans laquelle sont formés des deuxièmes supports (72 et 71) diamétralement opposés pour ces organes (16 et 17).

2. Système selon la revendication 1, caractérisé en ce que le premier circuit électronique (18) comprend :
- un premier bloc d'amplification (36) pour amplifier le deuxième signal,
- une boucle de rétroaction pour le premier bloc d'amplification (36), comprenant un dispositif électronique (41) pour commander automatiquement le gain du premier bloc d'amplification (36) et produire à sa sortie le troisième signal de tension alternatif d'amplitude constante,
- un bloc de rectification (37) pour convertir le troisième signal en un quatrième signal de tension continue, et
- un deuxième bloc d'amplification (38) pour comparer le quatrième signal avec une première tension de référence (+V1), générant un quatrième signal de tension continue proportionnellement au résultat de la comparaison, et contrôler le dispositif (41) à travers le quatrième signal.

3. Système selon la revendication 2, caractérisé en ce que le dispositif électronique (41) comprend un optocoupleur ayant une photorésistance (43) et une photodiode (42).

4. Système selon la revendication 3, caractérisé en ce que :
- le premier bloc d'amplification (36) présente une première entrée pour recevoir le deuxième signal, une sortie connectée à l'entrée du bloc de rectification (37), et une deuxième entrée mise à la terre via une première résistance (44) et connectée à sa sortie à travers la photorésistance (43),
- le bloc de rectification (37) présente sa sortie connectée à une première entrée du deuxième bloc d'amplification (38), et
- le deuxième bloc d'amplification (38) présente une seconde entrée connectée à la première tension de référence (+V1), et sa sortie connectée à la terre via une deuxième résistance (45) et la photodiode (42).

5. Système selon l'une des revendications précédentes, caractérisé en ce que le deuxième circuit (22) comprend, en série, un premier bloc de comparaison (46), un bloc d'intégration (47) et un deuxième bloc de comparaison (48).

6. Système selon la revendication 5, caractérisé en ce que :
- le premier bloc de comparaison (46) présente une première entrée connectée via le réseau (52) à la sortie du deuxième bloc d'amplification (38), une deuxième entrée connectée à une deuxième tension de référence continue (+V3), et sa sortie connectée à l'entrée du bloc d'intégration (47),
- le deuxième bloc de comparaison (48) présente une première entrée connectée à la sortie du bloc d'intégration (47), une deuxième entrée connectée à une troisième tension de référence continue (-V4), et sa sortie connectée à l'entrée de l'unité (13).

7. Système selon la revendication 6, caractérisé en ce que le bloc d'intégration (47) comprend deux branches parallèles, dont la première présente une troisième résistance (91), et dont la deuxième présente une diode (92) et une quatrième résistance (93) ayant une valeur plus basse que la troisième résistance (91), les deux branches étant mises à la terre via le condensateur (94).

8. Système selon la revendication 7, caractérisé en ce que le bloc d'intégration (47) permet l'intégration asymétrique du signal généré par le premier bloc de comparaison (46), et la production aux bornes du condensateur (94) d'une tension proportionnelle à la longueur du signal généré par le premier bloc de comparaison (46), lorsqu'une période de temps prédéterminée plus grande que le temps de décharge du condensateur (94) s'est écoulée entre un signal et le suivant, ou proportionnelle à la somme des impulsions plus courte que cette période de temps, pourvu que les impulsions soient très proches les unes des autres, le bloc d'intégration (47) permettant dans ce dernier cas le comptage des bulles à travers la chambre à gouttes (7), le passage d'un nombre de bulles successives de petit volume étant calculé comme celui d'une seule bulle de plus grand volume.

9. Système selon l'une des revendications 2 à 8, caractérisé en ce qu'il comprend un troisième bloc de comparaison (21) pour comparer le quatrième signal avec une quatrième tension de référence (+V2), pour, au cas où le niveau de fluide dans la chambre à gouttes (7) deviendrait inférieur à l'axe des organes (16 et 17), déterminer une valeur proche de zéro du quatrième signal, ce quatrième signal qui contrôle le dispositif (41) tendant vers une valeur élevée en comparaison, et définissant à la sortie du troisième bloc de comparaison (21) un sixième signal qui est envoyé à l'unité (13) pour arrêter le traitement de dialyse.

10. Système selon l'une des revendications précédentes, caractérisé en ce qu'il comprend en amont du premier organe (16), un bloc de commutation (31) connecté ou déconnecté par un signal logique (test) transmis par une ligne (32) depuis l'unité (13), le bloc de commutation (31), lorsqu'il est déconnecté, permettant au premier signal d'alimenter complètement le premier organe (16), et quand il est connecté, d'atténuer une partie prédéterminée du premier signal pour simuler la présence d'une quantité prédéterminée d'air, et de la sorte, un signal d'alarme de bulles prédéterminé, pour détecter tout composant défectueux du système et permettre à l'unité (13) d'arrêter le fonctionnement de l'unité de dialyse (1).

11. Système selon l'une des revendications précédentes, caractérisé en ce qu'il comprend un premier actuateur (23) pour mettre en route une unité de pompage (12) faisant partie du circuit hydraulique de l'unité (1), un deuxième actuateur (29) pour mettre en route l'alimentation en fluide depuis la chambre à gouttes (7) à travers un dispositif de préhension (84), et des indicateurs d'alarme lumineux et/ou acoustiques (25 et 26), les actuateurs (23 et 29) et les indicateurs (25 et 26) étant commandés par l'unité (13).

12. Système selon l'une des revendications précédentes, caractérisé en ce que le corps (6) est formé en deux parties, dont une première partie (61) est fixe et dont une deuxième partie (62) est conçue pour être déplacée au moyen d'un levier opéré manuellement à l'encontre de l'action d'un ressort (82), entre une première position dans laquelle au moyen du ressort (82), la chambre à gouttes (7) est fixée entre les deux parties (61 et 62), et une deuxième position où la deuxième partie (62) est placée à une distance prédéterminée de l'autre, permettant ainsi le retrait de la chambre à gouttes (7) du premier support (67).

13. Système selon la revendication 12, caractérisé en ce que les parties (61 et 62) présentent une face latérale respective qui est formée en évidement semi-cylindrique respectif, ces évidements définissant le premier support (67).

14. Système selon la revendication 13, caractérisé en ce que la première partie (61) présente un troisième support (64) pour y loger un réservoir (8), et des évidements (65) pour supporter des conduites (11), et un troisième support (64) présentant un prolongement intérieur (66) permettant seulement l'insertion des réservoirs (8) et présentant un évidement compatible avec ce prolongement (66).
